# FASCICULE DE BREVET EUROPEEN

(11) **EP 0 895 472 B1**
(45) Date de publication et mention de la délivrance du brevet: **10.03.2004**
(21) Numéro de dépôt: 97918210.2
(22) Date de dépôt: 11.04.1997
(51) Int. Cl.: A61K 7/13

(54) **PROCEDE DE TEINTURE DES FIBRES KERATINIQUES AVEC DES PRECURSEURS DE COLORANTS D'OXYDATION ET DES COLORANTS DIRECTS EN POUDRE**
VERFAHREN ZUR FÄRBUNG VON KERATINISCHEN FASERN MIT OXIDATIONSFARBSTOFFVORPRODUKTEN UND PULVERFÖRMIGEN DIREKTFARBSTOFFEN
METHOD FOR DYEING KERATIN FIBRES WITH OXIDATION DYE PRECURSORS AND DIRECT POWDER DYES

(30) Priorité: 25.04.1996 FR 9605252
(43) Date de publication de la demande: 10.02.1999
(73) Titulaire: L'OREAL, 75008 Paris (FR)
(72) Inventeur: RONDEAU, Christine, F-78500 Sartrouville (FR); ZEMORI, Nicole, F-93260 Les Lilas (FR)
(74) Mandataire: Miszputen, Laurent
(86) Numéro de dépôt international: PCT/FR1997/000650
(87) Numéro de publication internationale: WO 1997/039727

(56) Documents cités:
- WO-A-95/01772
- DE-A- 3 814 685
- GB-A- 2 093 867
- GB-A- 2 093 868
- GB-A- 2 192 645

## Description

L'invention concerne un procédé de teinture des fibres kératiniques, en particulier des fibres kératiniques humaines, et notamment les cheveux, caractérisé par le fait qu'on applique sur les fibres kératiniques, au moment de l'emploi, un mélange extemporané : d'une composition (A) contenant au moins un précurseur de colorant d'oxydation et éventuellement au moins un coupleur, d'une composition (B), en poudre, contenant au moins un colorant direct, et d'une composition (C) contenant au moins un agent oxydant.

L'utilisation de précurseurs de colorants d'oxydation est largement répandue dans le domaine de la coloration capillaire. Cette classe de colorants comprend des composés initialement peu ou pas colorés appelés couramment "bases d'oxydation", qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, appelés couramment "coupleurs", et généralement présents dans les compositions tinctoriales utilisées en teinture d'oxydation.

La variété des molécules mises en jeu, qui sont constituées d'une part par les "bases d'oxydation" et d'autre part par les "coupleurs", permet l'obtention d'une palette très riche en coloris.

Pour varier encore les nuances obtenues et leur donner du reflet, il est bien connu également, d'utiliser en association avec des précurseurs de colorants d'oxydation et des coupleurs, des colorants directs, c'est-à-dire des substances colorées qui apportent une coloration en l'absence d'agent oxydant.

Cependant l'utilisation classique, c'est-à-dire dans une même composition tinctoriale, des précurseurs de colorants d'oxydation et des colorants directs, comme, en particulier la plupart des nitrés benzéniques, est limitée, par le fait que ces colorants directs sont particulièrement réactifs vis-à-vis des agents réducteurs qu'il faut ajouter généralement dans les compositions contenant des précurseurs de colorants d'oxydation pour empêcher l'oxydation prématurée desdits précurseurs avant le moment choisi pour le développement de la coloration sur le cheveu, par exemple au cours du stockage.

Cette réactivité vis-à-vis des réducteurs se traduit par une perte progressive ou une modification du pouvoir tinctorial des colorants directs au cours du stockage des compositions tinctoriales avant leur utilisation.

En outre, l'utilisation classique des colorants directs est limitée en concentration dans la composition pour des raisons de solubilité desdits colorants dans le support de teinture. Il en résulte que la puissance tinctoriale des compositions obtenues est souvent limitée.

Pour pallier à ces inconvénients, la demanderesse a effectué de nombreuses recherches sur la question, et vient maintenant de découvrir, de manière surprenante, qu'il est possible d'utiliser des colorants directs à des concentrations plus élevées que dans l'art antérieur, et d'obtenir des teintures puissantes, présentant en outre une bonne résistance vis-à-vis des agents atmosphériques tels que la lumière et les intempéries, et vis-à-vis de la transpiration et des différents traitements que peuvent subir les cheveux (lavages, déformations permanentes), en utilisant au moment de l'emploi, un mélange extemporané de trois composants (A), (B), (C), préalablement conditionnés séparément, dans lesquels (A) contient le précurseur de colorant d'oxydation et éventuellement le coupleur, (B) contient le colorant direct, en poudre, ou dispersé dans un excipient organique et/ou un excipient pulvérulent minéral sous forme de poudre, et (C), l'agent oxydant.
Cette invention permet également de mieux conserver le pouvoir tinctorial des compositions.

Cette découverte est à la base de la présente invention.

La présente invention a ainsi pour objet un procédé de teinture des fibres kératiniques, en particulier des fibres kératiniques humaines, et notamment les cheveux, caractérisé par le fait qu'on applique sur les fibres kératiniques, au moment de l'emploi, un mélange extemporané de trois compositions (A), (B), et (C) suivantes :
- une composition (A) contenant au moins un précurseur de colorant d'oxydation et éventuellement au moins un coupleur, dans un milieu approprié pour la teinture,
- une composition (B), sous forme de poudre, contenant au moins un colorant direct, éventuellement dispersé dans un excipient pulvérulent organique et/ou un excipient pulvérulent minéral, et,
- une composition (C) contenant au moins un agent oxydant dans un milieu approprié pour la teinture.

L'invention a également pour objet une composition, prête à l'emploi, à trois composants (A), (B) ét (C), stockés de façon séparée et mélangés au moment de l'emploi, pour l'application sur les fibres kératiniques.

Un autre objet de l'invention concerne des dispositifs à plusieurs compartiments ou "kits", pour la teinture des fibres kératiniques, caractérisés par le fait qu'ils comportent au moins trois compartiments, dont l'un d'eux renferme une composition (A) contenant au moins un précurseur de colorant d'oxydation et éventuellement au moins un coupleur, dans un milieu approprié pour la teinture, un deuxième une composition (B), sous forme de poudre, contenant au moins un colorant direct, en poudre, ou dispersé dans un excipient pulvérulent organique et/ou un excipient pulvérulent minéral, et un troisième une composition (C) contenant au moins un agent oxydant dans un milieu approprié pour la teinture.

Mais d'autres caractéristiques, aspects, objets et avantages de l'invention apparaîtront encore plus clairement à la lecture de la description et des exemples qui suivent.

Les précurseurs de colorants d'oxydation utilisables dans le procédé de teinture selon l'invention sont ceux classiquement utilisés dans les compositions de teinture d'oxydation, c'est-à-dire des ortho- ou paraphénylènediamines, des bis-phénylalkylènediamines, des ortho- ou paraaminophénols, ou encore des bases hétérocycliques, ainsi que les sels d'addition de ces composés avec un acide.

Parmi les paraphénylènediamines utilisables à titre de bases d'oxydation dans le procédé de teinture selon l'invention, on peut notamment citer les composés répondant à la formule (I) suivante, et leurs sels d'addition avec un acide : 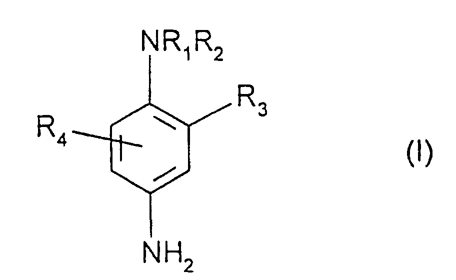 dans laquelle :
R₁ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₂ représente un atome d'hydrogène, un radical alkyle en C₁-C₄, monohydroxyalkyle en C₁-C₄ ou polyhydroxyalkyle en C₂-C₄,
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₄ représente un atome d'hydrogène ou un radical alkyle en C₁-C₄.

Dans la formule (I) ci-dessus, et lorsque R₃ est différent d'un atome d'hydrogène, alors R₁ et R₂ représentent de préférence un atome d'hydrogène et R₃ est de préférence identique à R₄, et lorsque R₃ représente un atome d'halogène, alors R₁, R₂ et R₄ représentent de préférence un atome d'hydrogène.

Parmi les paraphénylènediamines de formule (I) ci-dessus, on peut plus particulièrement citer la paraphénylènediamine, la paratoluylènediamine, la 2-isopropyl paraphénylènediamine, la 2-(β-hydroxyéthyl) paraphénylènediamine, la 2-(β-hydroxyéthyloxy) paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans le procédé de teinture selon l'invention, on peut notamment citer les composés répondant à la formule (II) suivante, et leurs sels d'addition avec un acide : 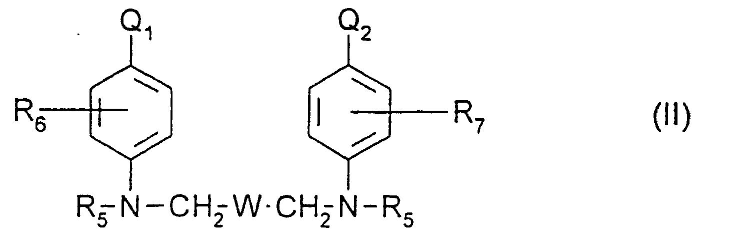 dans laquelle :
Q₁ et Q₂, identiques ou différents, représentent un radical hydroxyle ou NHR₈ dans lequel R₈ représente un atome d'hydrogène ou un radical alkyle en C₁-C₄,
R₅ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₆ et R₇, identiques ou différents, représentent un atome d'hydrogène ou d'halogène ou un radical alkyle en C₁-C₄,
W représente un radical pris dans le groupe constitué par les radicaux suivants :

   -(CH₂)ₙ ;

   -(CH₂)ₘ-O-(CH₂)ₘ ;

   -(CH₂)ₘ-CHOH-(CH₂)ₘ

   et 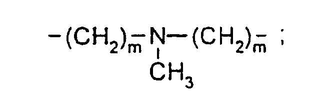
dans lesquels n est un nombre entier compris entre 0 et 8 inclusivement et m est un nombre entier compris entre 0 et 4 inclusivement.

Parmi les bis-phénylalkylènediamines de formules (II) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (II), le N,N'-bis-(β-hydroxyéthyl) N,N'-bis-(4'-aminophényl) 1,3-diamino 2-propanol ou l'un de ses sels d'addition avec un acide sont particulièrement préférés.

Parmi les para-aminophénols utilisables à titre de bases d'oxydation dans le procédé de teinture selon l'invention, on peut notamment citer les composés répondant à la formule (III) suivante, et leurs sels d'addition avec un acide : 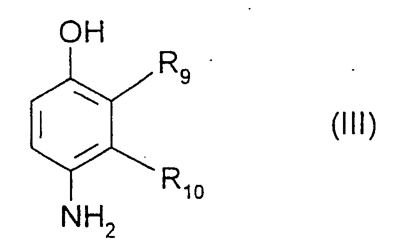 dans laquelle :
R₉ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₀ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₉ ou R₁₀ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthylaminométhyl) phénol, et leurs sels d'addition avec un acide.

Parmi les ortho-aminophénols utilisables à titre de bases d'oxydation dans le procédé de teinture selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans le procédé de teinture selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1 026 978 et GB-1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE-2 359 399 ou japonais JP-88-169571 et JP-91-333 495, comme la 2,4,5,6-tétra-aminopyrimidine, la 4-hydroxy 2,5,6-triaminopyrimidine, et leurs sels d'addition avec un acide.

Parmi les dérivés pyrazoliques, on peut plus particulièrement citer les composés décrits dans les brevets DE-3 843 892, DE-4 133 957 et demandes de brevet WO-94/08969 et WO-94/08970 comme le 4,5-diamino-1-méthyl-pyrazole, le 3,4-diamino-pyrazole, et leurs sels d'addition avec un acide.

Selon l'invention, la ou les bases d'oxydation représentent de préférence de 0,0005 à 12% en poids environ du poids total de la composition (A), et encore plus préférentiellement de 0,005 à 6% en poids environ.

Les coupleurs utilisables dans le procédé de teinture selon l'invention sont ceux classiquement utilisés dans les compositions de teinture d'oxydation, c'est à dire des métaphénylènediamines, des métaaminophénols et des métadiphénols, et des coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le 1,3-bis-[(4-aminophényl) (2-hydroxyéthyl)-amino]-2-propanol, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H-3-méthyl pyrazole-5-one, le 1-phényl 3-méthyl pyrazole-5-one, et leurs sels d'addition avec un acide.

Lorsqu'ils sont présents, ces coupleurs représentent de préférence de 0,0001 à 10 % en poids environ du poids total de la composition (A), et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition (A) contient en outre et classiquement des agents réducteurs pour empêcher l'oxydation prématurée des précurseurs de colorants d'oxydation; lesdits réducteurs sont notamment et de manière connue, le bisulfite de sodium, l'acide thioglycolique, l'acide thiolactique et leurs sels. Il sont présents dans une proportion allant d'environ 0,05 à 3 % en poids et de préférence 0,1 à 1,5% en poids environ par rapport au poids total de la composition (A).

Les colorants directs utilisables dans le procédé de teinture selon l'invention sont ceux classiquement utilisés dans les compositions de teinture directe, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, mono- ou di-azoïques, aziniques, acridiniques et xanthéniques, ou encore des colorants métallifères.

Cependant, selon l'invention, on préfère plus particulièrement utiliser des colorants directs cationiques.

Parmi ceux-ci, on peut citer avantageusement le chlorure de 3-[(4-amino-6-bromo-5,8-dihydro-1-hydroxy-8-imino-5-oxo-2-naphtalényl)-amino]-N,N,N-triméthyl-benzénaminium (dénommé Basic Blue 99 dans le Color Index), ainsi que les colorants directs cationiques qui comportent un atome d'azote quaternisé éventuellement délocalisable et une liaison -Z=N- , dans laquelle Z désigne un atome d'azote ou un radical -CH- , et notamment ceux répondant à la formule (IV) suivante : 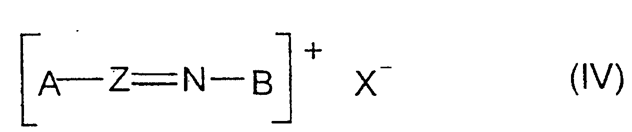 dans laquelle, Z désigne un atome d'azote ou un radical -CH- , A et B désignent des groupements aromatiques benzéniques ou hétérocycliques éventuellement substitués, de préférence par un ou plusieurs atomes d'halogène ou par un ou plusieurs radicaux tels que NR₁₁R₁₂, ou OR₁₁, dans lesquels R₁₁ et R₁₂, simultanément ou indépendamment l'un de l'autre, représentent l'hydrogène, un radical alkyle en C₁-C₈, un radical hydroxyalkyle en C₁-C₄, ou un radical phényle, X⁻ désigne un anion de préférence chlorure ou méthyl sulfate, la charge cationique étant portée par l'un des substituants du cycle A et/ou du cycle B.

Ces composés de formule (IV) sont par exemple décrits et préparés dans les demandes de brevets internationales WO-95/01772 et WO-95/15144 de la société CIBA-GEIGY.

Parmi les composés de formule (IV), on peut par exemple citer les composés de formules (10) à (14) suivantes : 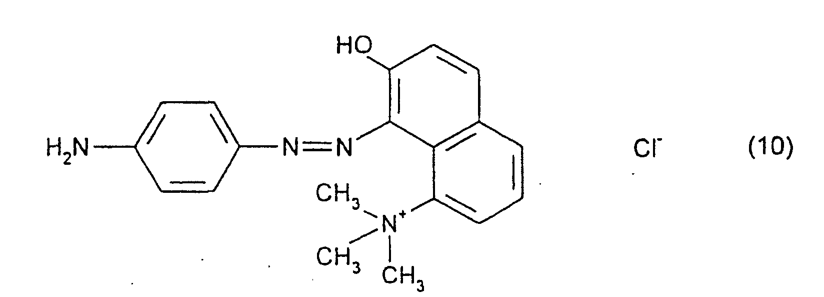 i.e. le chlorure de 4-amino-phényl-azo-2-hydroxy-8-triméthylammonionaphtalène ; 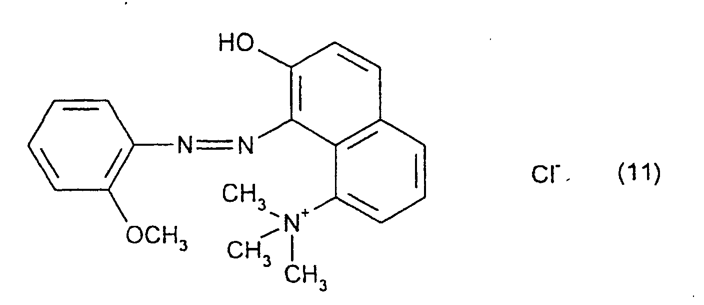 i.e. le chlorure de 2-méthoxy-phényl-azo-2-hydroxy-8-triméthylammonionaphtalène ; 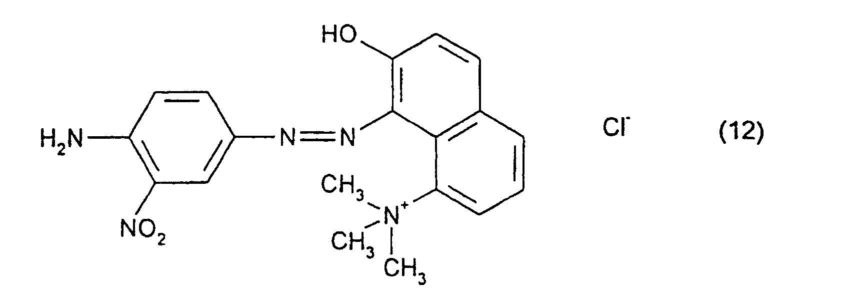 i.e. le chlorure de 4-amino-3-nitro-phényl-azo-2-hydroxy-8-triméthylammonionaphtalène, 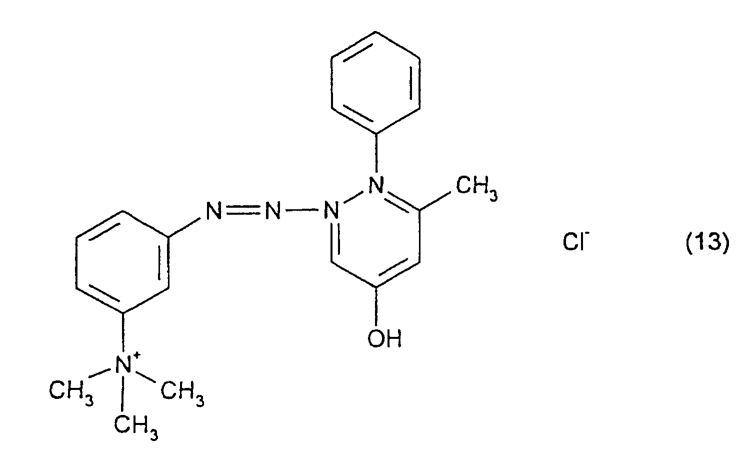 i.e. le chlorure de 3-triméthylammonio-phényl-azo-N-phényl-3-méthyl-5-hydroxy-pyridazine ; 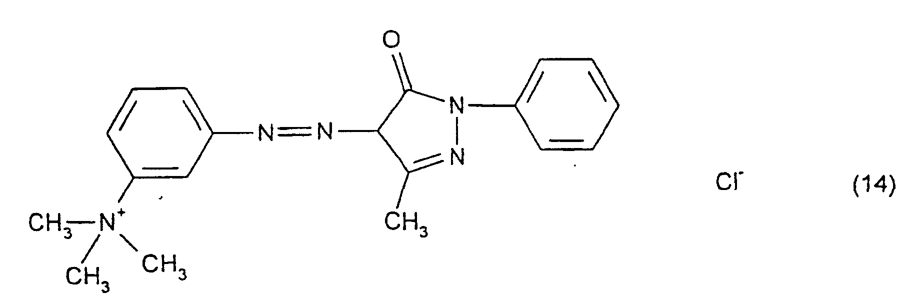 i.e. le chlorure de 3-[(4,5-dihydro-3-méthyl-5-oxo-1-phényl-1H-pyrazol-4-yl)-azo]-N,N,N-triméthyl-benzénaminium.

Les colorants directs représentent de préférence 0,1 à 100 % en poids environ du poids total de la composition (B) et plus avantageusement encore environ 1 à 50 % en poids.

Dans la composition (C), l'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition oxydante (C) est avantageusement constituée par une solution de peroxyde d'hydrogène dont le titre peut varier, plus particulièrement, d'environ 5 à 40 volumes.

Le milieu des compositions (A) et éventuellement (C), approprié pour la teinture, est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, par exemple, l'éthylèneglycol et ses éthers monométhylique, monoéthylique et monobutylique, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

Les compositions (A) et (C) peuvent encore contenir une quantité efficace d'autres agents, par ailleurs antérieurement connus en coloration d'oxydation, tels que des tensio-actifs anioniques, cationiques, non-ioniques, amphotères ou zwittérioniques ou leurs mélanges, des épaississants, et divers adjuvants usuels comme des séquestrants, des agents de conditionnement du cheveu, des conservateurs, des opacifiants, etc...

Dans la composition (B) en poudre, le colorant direct peut constituer à lui-seul toute la composition, ou être dispersé au sein d'un excipient, en poudre, de nature organique et/ou de nature minérale. Cette poudre présente de préférence une taille de particules inférieure à 350 µm.
L'excipient organique peut être d'origine synthétique ou végétale et choisi notamment parmi les polymères synthétiques réticulés ou non réticulés, les polysaccharides comme les celluloses et les amidons modifiés ou non ainsi que les produits naturels les renfermant tels que la sciure de bois, ou les gommes végétales (guar, caroube, xanthane, etc...).
L'excipient minéral peut être constitué par des oxydes métalliques tels que les oxydes de titane, les oxydes d'aluminium, le kaolin, le talc, les silicates, le mica et les silices.
Un excipient avantageusement préféré est constitué par de la sciure de bois.

La composition (B), en poudre, peut encore contenir des liants ou produits d'enrobage dans une quantité ne dépassant pas de préférence 3% en poids environ du poids total de ladite composition.
Ces liants sont de préférence des huiles ou corps gras liquides d'origine minérale, synthétique, animale ou végétale.
La composition (B), en poudre, peut éventuellement encore contenir d'autres adjuvants, à l'état de poudre, en particulier des tensio-actifs de toute nature, des agents de conditionnement du cheveu comme par exemple des polymères cationiques, etc...

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Les quantités relatives des compositions (A), (B) et (C), dans le procédé de teinture selon la présente invention, vont de préférence, et exprimées en parties en poids, d'environ 1 / 0,010 / 0,5, à 1 / 1 / 4 et encore plus préférentiellement de 1 / 0,05 / 0,5 à 1 / 0,5 / 2.

Le pH de la composition tinctoriale prête à l'emploi, à trois composants (A), (B) et (C), conforme à l'invention, qui est appliquée sur les fibres, est généralement compris entre 3 et 12. Il est de préférence compris entre les valeurs 8,5 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 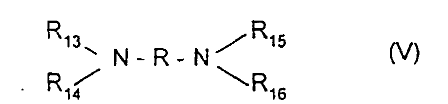 dans laquelle R est un reste propylène éventuellement substitué par un groupement hydroxyle ou un radical alkyle en C₁-C₄ ; R₁₃, R₁₄, R₁₅ et R₁₆, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi à partir des trois composants (A), (B) et (C) décrits ci-avant, sur les fibres kératiniques sèches ou humides, et à le laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Des exemples concrets illustrant l'invention vont maintenant être donnés, sans pour autant présenter un caractère limitatif.

### EXEMPLES

### EXEMPLE 1

### Composition (A) :

- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Monobutyléther de diéthylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Paraphénylènediamine 0,35 g
- 1,3-dihydroxybenzène 0,4 g
- 3-amino phénol 0, 03 g
- 2,4-diamino 1-(β-hydroxyéthyloxy) benzène, 2HCl 0,012 g
- 1,3-bis-[(4-aminophényl)(2-hydroxyéthyl)-amino]-2-propanol, 4HCl 0,037 g
- 1,3-dihydroxy 2-méthyl benzène 0,2 g
- Antioxydant, séquestrant q.s.
- Parfum, conservateur q.s.
- Ammoniaque à 20 % de NH₃ 10,0 g
- Eau déminéralisée q.s.p. 100 g

### Composition (B) :

- Colorant direct cationique de formule (11), en poudre 20 g
- Huile de paraffine 3 g
- Polymère cationique en poudre (Merquat 280 Dry de Calgon) 10 g
- Sciure de bois q.s.p. 100 g

### Composition (C) :

- Peroxyde d'hydrogène à 20 volumes 100 g

Au moment de l'emploi, on a mélangé 1 partie en poids de la composition (A) avec 0,1 partie en poids de la composition (B) et 1 partie en poids de la composition (C).

Le pH de la composition résultante était de 9,8.

On a alors appliqué cette composition sur des mèches de cheveux gris naturels ou permanentés à 90% de blancs. Après 30 minutes de pose, rinçage à l'eau courante, lavage avec un shampooing standard et séchage, les mèches ont été teintes dans une nuance blond foncé rouge, ayant une résistance satisfaisante vis-à-vis des agents atmosphériques, de la transpiration, et des différents traitements que peuvent subir les cheveux.

## Revendications

1. Procédé de teinture des fibres kératiniques, en particulier des fibres kératiniques humaines, et notamment les cheveux, **caractérisé par le fait qu'**on applique sur les fibres kératiniques, au moment de l'emploi, un mélange extemporané de 3 compositions (A), (B), et (C) suivantes :
- une composition (A) contenant au moins un précurseur de colorant d'oxydation et éventuellement au moins un coupleur, dans un milieu approprié pour la teinture,
- une composition (B), sous forme de poudre, contenant au moins un colorant direct, éventuellement dispersé dans un excipient pulvérulent organique et/ou un excipient pulvérulent minéral, et,
- une composition (C) contenant au moins un agent oxydant dans un milieu approprié pour la teinture.

2. Procédé de teinture selon la revendication 1, **caractérisé par le fait que** dans la composition (B) en poudre, le colorant direct est choisi parmi les colorants nitrés benzéniques, les nitropyridines, les colorants anthraquinoniques, les colorants mono- ou di-azoïques, aziniques, acridiniques et xanthéniques, ou encore les colorants métallifères.

3. Procédé de teinture selon la revendication 1, **caractérisé par le fait que**, dans la composition (B) en poudre, le colorant direct est un colorant cationique.

4. Procédé de teinture selon la revendication 3, **caractérisé par le fait que** le colorant est le chlorure de 3-[(4-amino-6-bromo-5,8-dihydro-1-hydroxy-8-imino-5-oxo-2-naphtalényl)-amino]-N,N,N-triméthyl-benzénaminium.

5. Procédé de teinture selon la revendication 3, **caractérisé par le fait que** le colorant comporte un atome d'azote quaternisé éventuellement délocalisable et une liaison -Z=N- , dans laquelle Z désigne un atome d'azote ou un radical -CH- .

6. Procédé de teinture selon la revendication 5, **caractérisé par le fait que** le colorant est un composé de formule (IV) suivante : 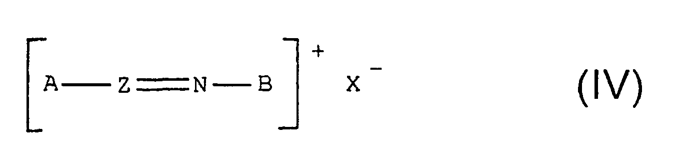 dans laquelle :
Z désigne un atome d'azote ou un radical -CH- , A et B désignent des groupements aromatiques benzéniques ou hétérocycliques éventuellement substitués, X⁻ désigne un anion, la charge cationique étant portée par l'un des substituants du cycle A et/ou du cycle B.

7. Procédé de teinture selon la revendication 6, **caractérisé par le fait que** le colorant est un composé choisi parmi les formules (10) à (14) suivantes : 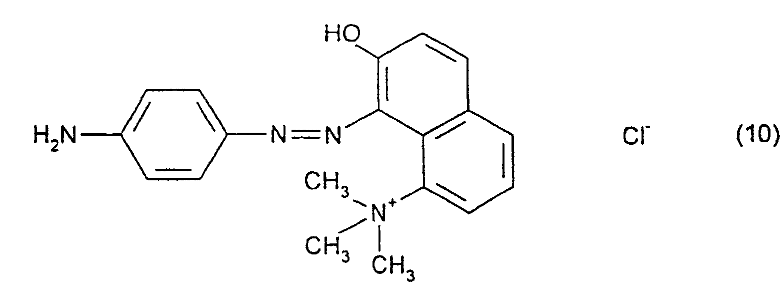 i.e. le chlorure de 4-amino-phényl-azo-2-hydroxy-8-triméthylammonionaphtalène ; 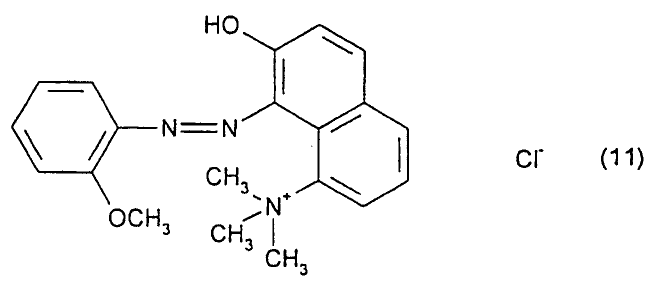 i.e. le chlorure de 2-méthoxy-phényl-azo-2-hydroxy-8-triméthylammonionaphtalène ; 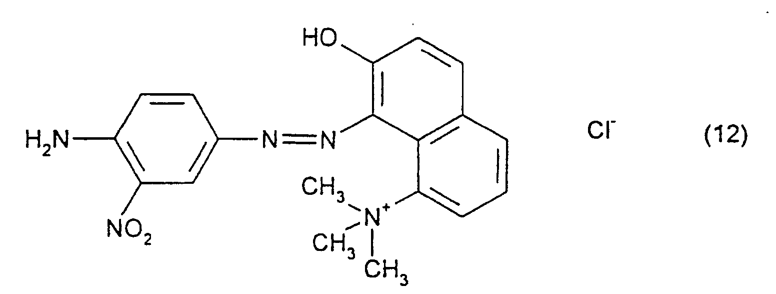 i.e. le chlorure de 4-amino-3-nitro-phényl-azo-2-hydroxy-8-triméthylammonionaphtalène, 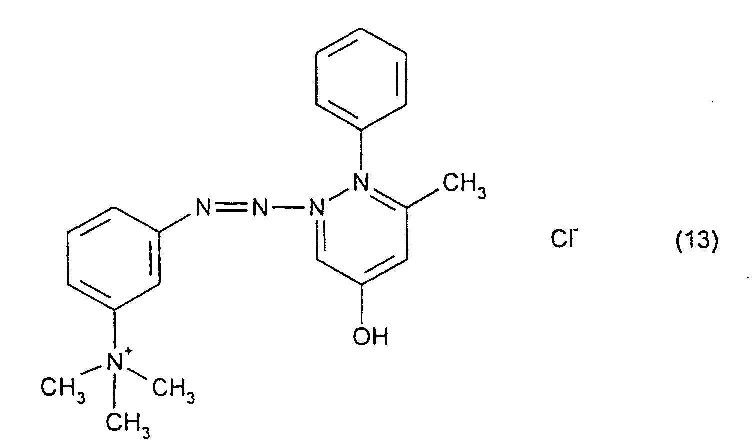 i.e. le chlorure de 3-triméthylammonio-phényl-azo-N-phényl-3-méthyl-5-hydroxy-pyridazine, 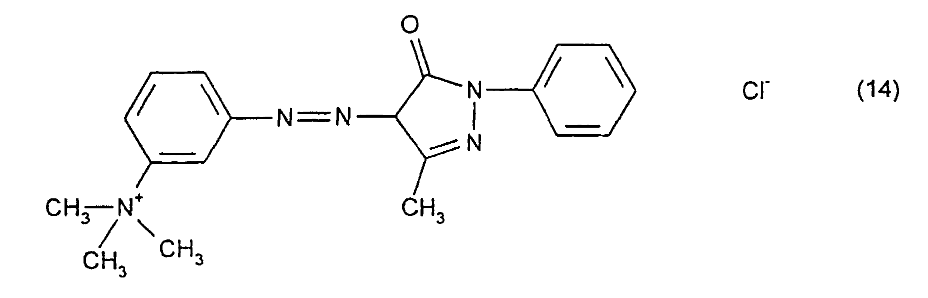 i.e. le chlorure de 3-[(4,5-dihydro-3-méthyl-5-oxo-1-phényl-1H-pyrazol-4-yl)-azo]-N,N,N-triméthyl-benzènaminium.

8. Procédé de teinture selon l'une quelconque des revendications précédentes, **caractérisé par le fait que** dans la composition (B) en poudre, le colorant direct est présent dans des concentrations allant de 0,1 à 100 % en poids du poids total de ladite composition.

9. Procédé de teinture selon l'une quelconque des revendications précédentes, **caractérisé par le fait que** dans la composition (A), les précurseurs de colorants d'oxydation sont choisis parmi les ortho- ou paraphénylènediamines, les bis-phénylalkylènediamines, les ortho- ou para-aminophénols, les bases hétérocycliques, et leurs sels d'addition avec un acide.

10. Procédé de teinture selon l'une quelconque des revendications précédentes, **caractérisé par le fait que** dans la composition (A), les précurseurs de colorants d'oxydation sont présents dans des concentrations allant de 0,0005 à 12 % en poids par rapport au poids total de ladite composition.

11. Procédé de teinture selon l'une quelconque des revendications précédentes, **caractérisé par le fait que** dans la composition (A), les coupleurs sont choisis parmi les métaphénylènediamines, les métaaminophénols, les métadiphénols, les coupleurs hétérocycliques, et leurs sels d'addition avec un acide.

12. Procédé de teinture selon l'une quelconque des revendications précédentes, **caractérisé par le fait que** dans la composition (A), les coupleurs sont présents dans des concentrations allant de 0,0001 à 10 % en poids par rapport au poids total de ladite composition.

13. Procédé de teinture selon l'une quelconque des revendications précédentes, **caractérisé par le fait que** dans la composition (A), les sels d'addition avec un acide des précurseurs de colorants d'oxydation et des coupleurs sont choisis parmi les chlorhydrates, les bromhydrates, les sulfates, les tartrates, les lactates et les acétates.

14. Procédé de teinture selon l'une quelconque des revendications précédentes, **caractérisé par le fait que** la composition (A) contient en outre au moins un agent réducteur.

15. Procédé de teinture selon revendication 14, **caractérisé par le fait que** dans la composition (A), l'agent réducteur est choisi parmi le bisulfite de sodium, l'acide thioglycolique, l'acide thiolactique et leurs sels, et est présent dans une concentration allant de 0,05 à 3 % en poids par rapport au poids total de ladite composition.

16. Procédé de teinture selon l'une quelconque des revendications précédentes, **caractérisé par le fait que** dans la composition (C), l'agent oxydant est choisi parmi le peroxyde d'hydrogène, le peroxyde d'urée, les bromates de métaux alcalins et les persels.

17. Procédé de teinture selon la revendication 16, **caractérisé par le fait que** l'agent oxydant est une solution de peroxyde d'hydrogène dont le titre varie de 5 à 40 volumes.

18. Procédé de teinture selon. l'une quelconque des revendications précédentes, **caractérisé par le fait que** dans la composition (B) en poudre, le colorant direct est dispersé dans un excipient pulvérulent de nature organique et/ou dans un excipient pulvérulent de nature minérale.

19. Procédé de teinture selon la revendication 18, **caractérisé par le fait que** l'excipient est constitué par de la sciure de bois.

20. Procédé de teinture selon l'une quelconque des revendications précédentes, **caractérisé par le fait que** les quantités relatives des compositions (A), (B) et (C), exprimées en parties en poids, varient de 1 / 0,010 / 0,5 à 1 / 1 / 4.

21. Dispositif à plusieurs compartiments ou "kits", pour la teinture des fibres kératiniques, **caractérisé par le fait qu'**il comporte au moins trois compartiments, dont l'un d'eux renferme une composition (A) telle que définie dans les revendications 1, 9 à 15, contenant au moins un précurseur de colorant d'oxydation et éventuellement au moins un coupleur, dans un milieu approprié pour la teinture, un deuxième une composition (B) telle que définie dans les revendications 1 à 8, 18 et 19, sous forme de poudre, contenant au moins un colorant direct, en poudre, ou dispersé dans un excipient pulvérulent organique et/ou un excipient pulvérulent minéral, et un troisième une composition (C) telle que définie dans les revendications 1, 16, 17, contenant au moins un agent oxydant dans un milieu approprié pour la teinture.

## Patentansprüche

1. Verfahren zum Färben von Keratinfasern und insbesondere menschlichen Keratinfasern, besonders zum Färben der Haare, **dadurch gekennzeichnet, dass** auf die Keratinfasern bei der Anwendung ein bedarfsgemäß hergestelltes Gemisch aus den drei folgenden Zusammensetzungen (A), (B) und (C) aufgetragen wird:
- eine Zusammensetzung (A), die mindestens ein Farbstoffvorprodukt eines Oxidationsfarbstoffes und gegebenenfalls mindestens einen Kuppler in einem zum Färben geeigneten Medium enthält,
- eine Zusammensetzung (B) in Pulverform, die mindestens einen Direktfarbstoff enthält, der gegebenenfalls in einem organischen pulverförmigen Träger und/oder einem anorganischen pulverförmigen Träger dispergiert ist, und
- eine Zusammensetzung (C), die mindestens ein Oxidationsmittel in einem zum Färben geeigneten Medium enthält.

2. Verfahren zum Färben nach Anspruch 1, **dadurch gekennzeichnet, dass** in der pulverförmigen Zusammensetzung (B) der Direktfarbstoff unter den Nitrobenzol-Farbstoffen, Nitropyridinen, Anthrachinon-Farbstoffen, Mono- oder Diazofarbstoffen, Azin-Farbstoffen, Acridin-Farbstoffen und Xanthen-Farbstoffen oder den metallhaltigen Farbstoffen ausgewählt ist.

3. Verfahren zum Färben nach Anspruch 1, **dadurch gekennzeichnet, dass** in der pulverförmigen Zusammensetzung (B) der Direktfarbstoff ein kationischer Farbstoff ist.

4. Verfahren zum Färben nach Anspruch 3, **dadurch gekennzeichnet, dass** der Farbstoff das 3-[(4-Amino-6-bromo-5,8-dihydro-1-hydroxy-8-imino-5-oxo-2-naphthyl)-amino]-N,N,N-trimethyl-benzolaminiumchlorid ist.

5. Verfahren zum Färben nach Anspruch 3, **dadurch gekennzeichnet, dass** der Farbstoff ein quaternisiertes Stickstoffatom mit gegebenenfalls delokalisierbarer Ladung und eine Bindung -Z=N- aufweist, worin Z ein Stickstoffatom oder die Gruppe -CH- bedeutet.

6. Verfahren zum Färben nach Anspruch 5, **dadurch gekennzeichnet, dass** der Farbstoff eine Verbindung der folgenden Formel (IV) ist: 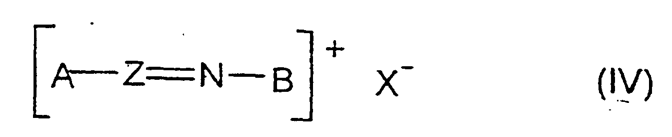 worin bedeuten:
Z ein Stickstoffatom oder die Gruppe -CH-, A und B aromatische Benzolgruppen oder heterocyclische Gruppen, die gegebenenfalls substituiert sind, X⁻ ein Anion, wobei sich die kationische Ladung an einem der Substituenten des Rings A und/oder des Rings B befindet.

7. Verfahren zum Färben nach Anspruch 6, **dadurch gekennzeichnet, dass** der Farbstoff eine Verbindung der folgenden Formeln (10) bis (14) ist: 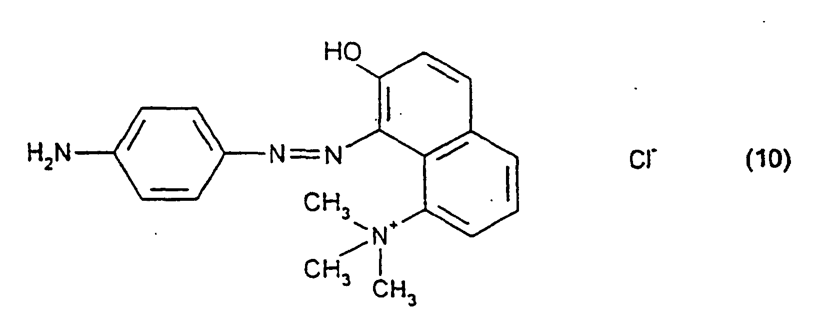 d.h. 4-Amino-phenyl-azo-2-hydroxy-8-trimethylammonionaphthalin-chlorid; 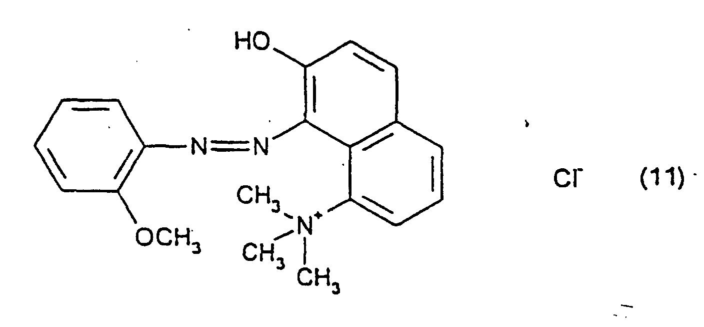 d.h. 2-Methoxy-phenyl-azo-2-hydroxy-8- trimethylammonionaphthalin-chlorid; 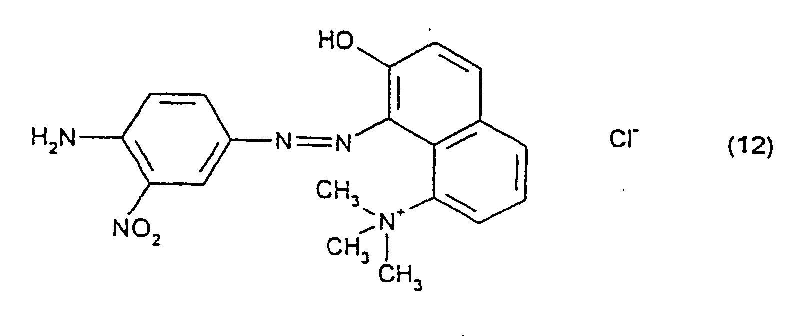 d.h. 4-Amino-3-nitro-phenyl-azo-2-hydroxy-8- trimethylammonionaphthalin-chlorid; 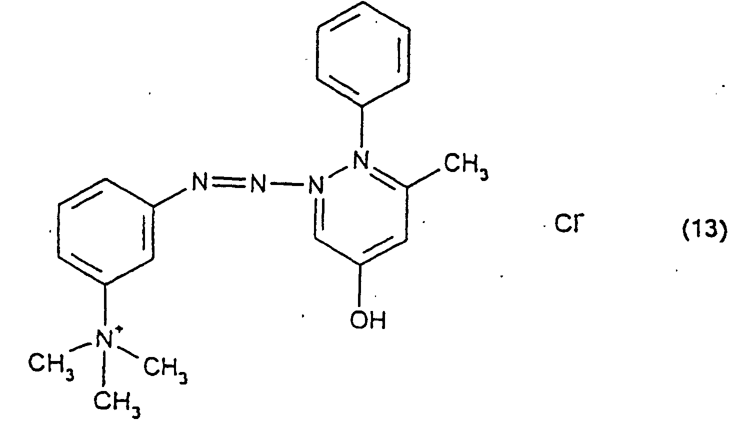 d.h. 3-Trimethylammonio-phenyl-azo-N-phenyl-3-methyl-5-hydroxypyridazin chlorid; 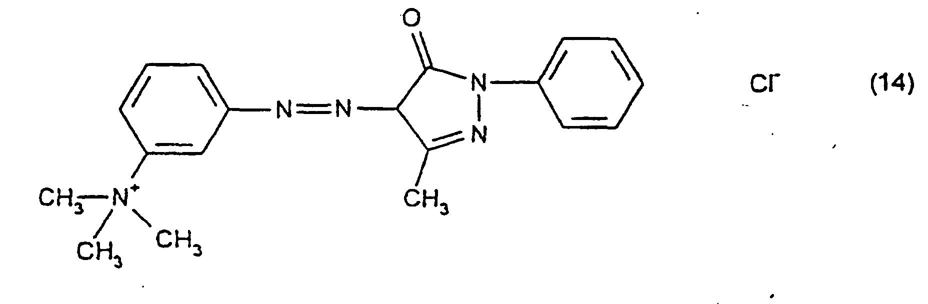 d.h. 3-[(4,5-Dihydro-3-methyl-5-oxo-1-phenyl-1H-pyrazol-4-yl)-azo]-N,N,N-trimethyl-benzolaminiumchlorid.

8. Verfahren zum Färben nach einem der vorhergehenden Ansprüche, **dadurch gekennzeichnet, dass** der Direktfarbstoff in der pulverförmigen Zusammensetzung (B) in Konzentrationen von 0,1 bis 100 Gew.-% des Gesamtgewichts der Zusammensetzung enthalten ist.

9. Verfahren zum Färben nach einem der vorhergehenden Ansprüche, **dadurch gekennzeichnet, dass** in der Zusammensetzung (A) die Farbstoffvorprodukte von Oxidationsfarbstoffen unter den o- oder p-Phenylendiaminen, Bisphenylalkylendiaminen, ortho- oder para-Aminophenolen, heterocyclischen Basen und deren Additionssalzen mit einer Säure ausgewählt sind.

10. Verfahren zum Färben nach einem der vorhergehenden Ansprüche, **dadurch gekennzeichnet, dass** in der Zusammensetzung (A) die Farbstoffvorprodukte von Oxidationsfarbstoffen in Konzentrationen von 0,0005 bis 12 Gew.-%, bezogen auf das Gesamtgewicht der Zusammensetzung, vorliegen.

11. Verfahren zum Färben nach einem der vorhergehenden Ansprüche, **dadurch gekennzeichnet, dass** die Kuppler in der Zusammensetzung (A) unter den m-Phenylendiaminen, m-Aminophenolen, m-Dihydroxybenzolen, heterocyclischen Kupplern und deren Additionssalzen mit einer Säure ausgewählt sind.

12. Verfahren zum Färben nach einem der vorhergehenden Ansprüche, **dadurch gekennzeichnet, dass** die Kuppler in der Zusammensetzung (A) in Konzentrationen von 0, 0001 bis 10 Gew.-%, bezogen auf das Gesamtgewicht der Zusammensetzung, enthalten sind.

13. Verfahren zum Färben nach einem der vorhergehenden Ansprüche, **dadurch gekennzeichnet, dass** die Additionssalze der Farbstoffvorprodukte von Oxidationsfarbstoffen und der Kuppler mit einer Säure in der Zusammensetzung (A) unter den Hydrochloriden, Hydrobromiden, Sulfaten, Tartraten, Lactaten und Acetaten ausgewählt sind.

14. Verfahren zum Färben nach einem der vorhergehenden Ansprüche, **dadurch gekennzeichnet, dass** die Zusammensetzung (A) ferner mindestens ein Reduktionsmittel enthält.

15. Verfahren zum Färben nach Anspruch 14, **dadurch gekennzeichnet, dass** in der Zusammensetzung (A) das Reduktionsmittel unter Natriumbisulfit, Thioglykolsäure, Thiomilchsäure und deren Salzen ausgewählt ist und in einer Konzentration von 0,05 bis 3 Gew.-%, bezogen auf das Gesamtgewicht der Zusammensetzung, vorliegt.

16. Verfahren zum Färben nach einem der vorhergehenden Ansprüche, **dadurch gekennzeichnet, dass** das Oxidationsmittel in der Zusammensetzung (C) unter Wasserstoffperoxid, Harnstoffperoxid, Alkalimetallbromaten und Salzen von Persäuren ausgewählt ist.

17. Verfahren zum Färben nach Anspruch 16, **dadurch gekennzeichnet, dass** das Oxidationsmittel eine Wasserstoffperoxidlösung mit einem Titer von 5 bis 40 Volumina ist.

18. Verfahren zum Färben nach einem der vorhergehenden Ansprüche, **dadurch gekennzeichnet, dass** der Direktfarbstoff in der pulverförmigen Zusammensetzung (B) in einem pulverförmigen Träger organischer Natur und/oder in einem pulverförmigen Träger anorganischer Natur dispergiert ist.

19. Verfahren zum Färben nach Anspruch 18, **dadurch gekennzeichnet, dass** der Träger aus Sägemehl besteht.

20. Verfahren zum Färben nach einem der vorhergehenden Ansprüche, **dadurch gekennzeichnet, dass** die relativen, in Gewichtsteilen ausgedrückten Mengen der Zusammensetzungen (A), (B) und (C) im Bereich von 1/0,010/0,5 bis 1/1/4 liegen.

21. Vorrichtung mit mehreren Abteilungen oder "Kits" zum Färben von Keratinfasern, **dadurch gekennzeichnet, dass** sie mindestens drei Abteilungen aufweist, wobei eine Abteilung eine Zusammensetzung (A) nach einem der Ansprüche 1, 9 bis 15 enthält, die mindestens ein Farbstoffvorprodukt eines Oxidationsfarbstoffes und gegebenenfalls mindestens einen Kuppler in einem zum Färben geeigneten Medium enthält, eine zweite Abteilung eine Zusammensetzung (B) nach einem der Ansprüche 1 bis 8, 18 und 19 in Pulverform enthält, die mindestens einen Direktfarbstoff in Pulverform oder einen in einem pulverförmigen organischen Träger und/oder in einem pulverförmigen anorganischen Träger dispergierten Direktfarbstoff enthält, und eine dritte Abteilung eine Zusammensetzung (C) nach einem der Ansprüche 1, 16 und 17 enthält, die mindestens ein Oxidationsmittel in einem zum Färben geeigneten Träger enthält.

## Claims

1. Process for dyeing keratin fibres, in particular human keratin fibres and especially the hair, **characterized in that** an extemporaneous mixture is applied to the keratin fibres at the time of use, this mixture comprising 3 compositions (A), (B) and (C) below:
- a composition (A) containing at least one oxidation dye precursor and optionally at least one coupler, in a medium which is suitable for dyeing,
- a composition (B) in powder form, containing at least one direct dye, optionally dispersed in an organic pulverulent excipient and/or an inorganic pulverulent excipient, and
- a composition (C) containing at least one oxidizing agent in a medium which is suitable for dyeing.

2. Dyeing process according to Claim 1, **characterized in that** in the powdered composition (B), the direct dye is chosen from nitrobenzene dyes, nitropyridine dyes, anthraquinone dyes, monoazo or diazo dyes, azine dyes, acridine dyes and xanthene dyes, or metalliferous dyes.

3. Dyeing process according to Claim 1, **characterized in that** in the powdered composition (B) the direct dye is a cationic dye.

4. Dyeing process according to Claim 3, **characterized in that** the dye is 3-[(4-amino-6-bromo-5,8-dihydro-1-hydroxy-8-imino-5-oxo-2-naphthalenyl)amino]-N,N,N-trimethylbenzenaminium chloride.

5. Dyeing process according to Claim 3, **characterized in that** the dye contains a quaternized nitrogen atom which is possibly delocalizable and a bond -Z=N-, in which Z denotes a nitrogen atom or a -CH- radical.

6. Dyeing process according to Claim 5, **characterized in that** the dye is a compound of formula (IV) below: 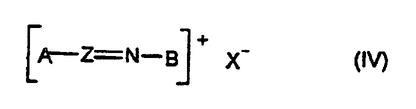 in which:
Z denotes a nitrogen atom or a -CH- radical, A and B denote benzenic or heterocyclic aromatic groups which are optionally substituted and X⁻ denotes an anion, the cationic charge being borne by one of the substituents on the ring A and/or on the ring B.

7. Dyeing process according to Claim 6, **characterized in that** the dye is a compound chosen from the formulae (10) to (14) below: 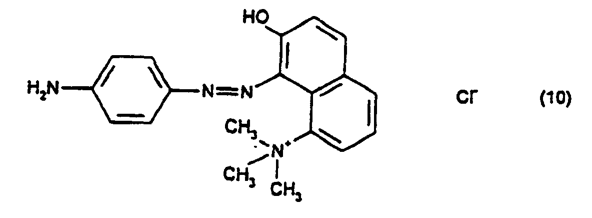 i.e. 4-aminophenylazo-2-hydroxy-8-trimethylammonionaphthalene chloride; 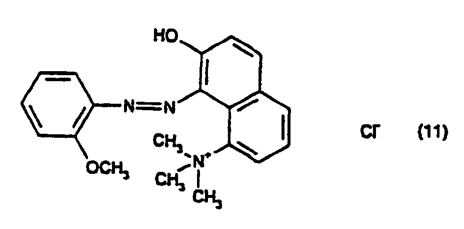 i.e. 2-methoxyphenylazo-2-hydroxy-8-trimethylammonionaphthalene chloride; 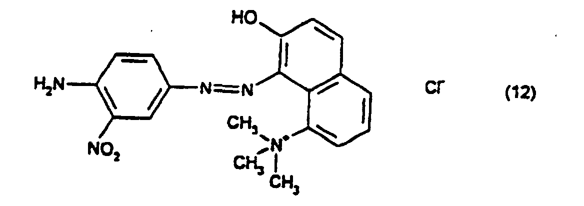 i.e. 4-amino-3-nitrophenylazo-2-hydroxy-8-trimethylammonionaphthalene chloride; 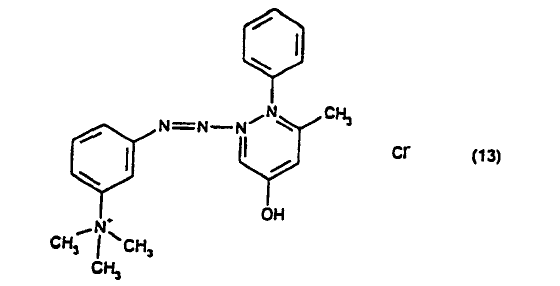 i.e. 3-trimethylammoniophenylazo-N-phenyl-3-methyl-5-hydroxypyridazine chloride; 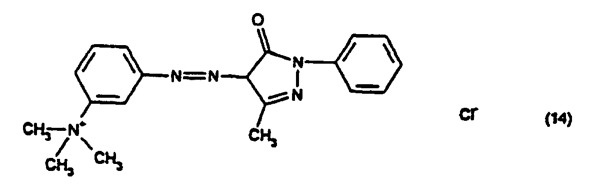 i.e. 3-[(4,5-dihydro-3-methyl-5-oxo-1-phenyl-1H-pyrazol-4-yl)azo]-N,N,N-trimethylbenzenaminium chloride.

8. Dyeing process according to any one of the preceding claims, **characterized in that** in the powdered composition (B), the direct dye is present in concentrations ranging from 0.1 to 100% by weight relative to the total weight of the said composition.

9. Dyeing process according to any one of the preceding claims, **characterized in that** in the composition (A), the oxidation dye precursors are chosen from ortho- or para-phenylenediamines, bis(phenyl)alkylenediamines, ortho- or para-aminophenols and heterocyclic bases, and the addition salts thereof with an acid.

10. Dyeing process according to any one of the preceding claims, **characterized in that** in the composition (A), the oxidation dye precursors are present in concentrations ranging from 0.0005 to 12% by weight relative to the total weight of the said composition.

11. Dyeing process according to any one of the preceding claims, **characterized in that** in the composition (A), the couplers are chosen from metaphenylenediamines, meta-aminophenols, meta-diphenols and heterocyclic couplers, and the addition salts thereof with an acid.

12. Dyeing process according to any one of the preceding claims, **characterized in that** in the composition (A), the couplers are present in concentrations ranging from 0.0001 to 10% by weight relative to the total weight of the said composition.

13. Dyeing process according to any one of the preceding claims, **characterized in that** in the composition (A), the addition salts with an acid of the oxidation dye precursors and of the couplers are chosen from the hydrochlorides, hydrobromides, sulphates, tartrates, lactates and acetates.

14. Dyeing process according to any one of the preceding claims, **characterized in that** the composition (A) also contains at least one reducing agent.

15. Dyeing process according to Claim 14, **characterized in that** in the composition (A), the reducing agent is chosen from sodium bisulphite, thioglycolic acid and thiolactic acid, and the salts thereof, and is present in a concentration ranging from 0.05 to 3% by weight relative to the total weight of the said composition.

16. Dyeing process according to any one of the preceding claims, **characterized in that** in the composition (C), the oxidizing agent is chosen from hydrogen peroxide, urea peroxide, alkali metal bromates and persalts.

17. Dyeing process according to Claim 16, **characterized in that** the oxidizing agent is a hydrogen peroxide solution whose titre ranges from 5 to 40 volumes.

18. Dyeing process according to any one of the preceding claims, **characterized in that** in the powdered composition (B), the direct dye is dispersed in a pulverulent excipient of organic nature and/or in a pulverulent excipient of inorganic nature.

19. Dyeing process according to Claim 18, **characterized in that** the excipient consists of sawdust.

20. Dyeing process according to any one of the preceding claims, **characterized in that** the relative amounts of the compositions (A), (B) and (C), expressed in parts by weight, range from 1/0.010/0.5 to 1/1/4.

21. Multi-compartment device or "kits" for dyeing keratin fibres, **characterized in that** it contains at least three compartments, one of which contains a composition (A) as defined in Claims 1 and 9 to 15, containing at least one oxidation dye precursor and optionally at least one coupler, in a medium which is suitable for dyeing, a second contains a composition (B) as defined in Claims 1 to 8, 18 and 19, in powder form, containing at least one direct dye, in powder form or dispersed in an organic pulverulent excipient and/or an inorganic pulverulent excipient, and a third contains a composition (C) as defined in Claims 1, 16 and 17, containing at least one oxidizing agent in a medium which is suitable for dyeing.
